Europäisches Patentamt

European Patent Office

Office européen des brevets

(11) Numéro de publication: **0 310 470**
A1

## (12) DEMANDE DE BREVET EUROPEEN

(21) Numéro de dépôt: 88402236.9

(22) Date de dépôt: 06.09.88

(51) Int. Cl.⁴: **A 61 B 17/22**

(30) Priorité: 02.10.87 FR 8713618

(43) Date de publication de la demande:
05.04.89 Bulletin 89/14

(84) Etats contractants désignés:
AT BE CH DE GB IT LI NL SE

(71) Demandeur: EDAP INTERNATIONAL
Z.I. le Parc aux Vignes Rue des Vieilles Vignes
F-77200 Croissy Beaubourg Marnes la Vallée (FR)

(72) Inventeur: Dory, Jacques
91, Rue des Molveaux
F-77450 Coupvray (FR)

(74) Mandataire: Marquer, Francis et al
Cabinet Moutard 35, Avenue Victor Hugo
F-78960 Voisins le Bretonneux (FR)

(54) Procédé et dispositif de localisation et de destruction d'une anomalie anatomique au moyen d'ondes élastiques avec poursuite de la cible et déclenchement automatique des tirs.

(57) Dispositif de localisation et de traitement d'une cible anatomique comportant l'émission périodique (1) d'un faisceau focalisé fixe d'ondes élastiques de traitement, la formation d'une image échographique en temps réel de la cible entre les émissions pendant la période de traitement, au moyen d'un faisceau auxiliaire d'ultrasons (2) effectuant un balayage sensiblement axé dans un plan de symétrie du faisceau fixe, l'affichage sur le support de visualisation (40) des images d'un repère représentant la position théorique du foyer du faisceau fixe et le déplacement solidaire des sources d'émission du faisceau jusqu'à coïncidence du repère avec l'image de la cible, caractérisé par la sélection (3-15) des échos de formation de l'image de la cible en fonction des coordonnées de la région d'impact du faisceau échographique par rapport à la source échographique, par l'asservissement (21-23) dudit déplacement à partir de signaux dérivés des échos sélectionnés jusqu'à obtention de ladite coïncidence et par le déclenchement automatique (1001) des tirs lorsque cette coïncidence est réalisée.

FIG.1

Description

## PROCEDE ET DISPOSITIF DE LOCALISATION ET DE DESTRUCTION D'UNE ANOMALIE ANATOMIQUE AU MOYEN D'ONDES ELASTIQUES, AVEC POURSUITE DE LA CIBLE ET DECLENCHEMENT AUTOMATIQUE DES TIRS.

En lithotripsie extracorporelle ou, a fortiori, en hyperthermie ou traitement des cellules malades par ondes élastiques, le problème de la localisation de la cible présente des difficultés : si les tirs (ondes élastiques de puissance, assurant la destruction de la cible, soit par chauffage, soit par contraintes mécaniques) peuvent facilement être focalisés au moyen d'un système acoustique comportant une lentille, un réflecteur ou une surface émissive ayant un foyer, les différences inévitables de vitesse de propagation des ondes élastiques entre le milieu de couplage et le corps du patient, de structure non homogène, provoquent des phénomènes de réfraction qui font que la position réelle du foyer à l'intérieur du corps n'est pas déterminable avec précision à partir de la connaissance de la position théorique, telle qu'elle est définie par le système acoustique.

On a proposé différents procédés de localisation du foyer et de la cible. Ceux qui font appel à des rayons X ont l'inconvénient, outre leur caractère destructif, de ne pas permettre par exemple la localisation de certains calculs biliaires, qui sont transparents aux rayons X. L'échographie par ultrasons est exempte de ces inconvénients, mais présente, a priori, un certain nombre de difficultés : la cible ne peut être atteinte qu'à travers une fenêtre acoustique plus ou moins étroite ; les signaux de destruction ont une fréquence trop basse pour l'échographie et, tout au moins en lithotripsie extracorporelle, une puissance trop élevée ; s'ils sont engendrés par une céramique piézoélectrique, celle-ci est de trop grandes dimensions pour être déplacée au rythme d'un balayage "temps réel" et de toutes façons, le foyer doit rester fixe pendant les périodes de traitement, ce qui exclut l'emploi du transducteur de puissance pour la localisation pendant le traitement proprement dit ; les procédés qui font appel à une échographie type A et à des mesures ou déterminations à l'aveugle ne sont pas suffisamment fiables pour assurer le contrôle de la localisation pendant les tirs, alors que ce contrôle est indispensable du fait que la cible, surtout s'il s'agit d'un calcul, va bouger au rythme de la respiration et peut même se déplacer brusquement. En hyperthermie, un contrôle "temps réel" est particulièrement indispensable et il doit être précis, si l'on veut éviter de détruire des cellules saines.

Dans le brevet européen N° 0 148 653 du 7 Novembre 1984, le Déposant a décrit un procédé de localisation visuelle qui consiste essentiellement à former des images échographiques en temps réel de la cible entre les tirs pendant la période de traitement, au moyen d'un faisceau auxiliaire d'ultrasons effectuant un balayage de préférence axé dans un plan de symétrie du faisceau focalisé principal de destruction, le plan de formation des images passant alors ainsi par le foyer du faisceau principal et à afficher, sur le support de visualisation de ces images, un repère représentant la position théorique dudit foyer, que l'opérateur amène en coïncidence avec l'image de la cible par déplacement de l'ensemble des deux sources qui émettent les faisceaux respectifs.

Ce procédé permet un contrôle visuel permanent et précis de la localisation pendant les tirs. Le brevet susvisé décrit également un perfectionnement important, qui permet de s'assurer à un moment quelconque que l'énergie ultrasonore de destruction est effectivement transmise au foyer et qu'elle est distribuée de façon homogène sur l'ensemble de la tache focale. A cet effet, il propose à titre de mesure complémentaire d'utiliser la source principale comme émetteur d'un faisceau échographique fixe et la source auxiliaire comme récepteur des échos formés par réflexion dudit faisceau fixe et de former ainsi une image de la tache focale correspondante en effectuant un balayage avec la source auxiliaire.

La présente invention a pour objet des perfectionnements aux procédés de localisation et de traitement, en particulier à ceux qui font l'objet du brevet susvisé.

Un premier perfectionnement autorise la poursuite de la cible et le déclenchement automatique des tirs quand elle est atteinte.

Suivant l'invention, ce résultat est obtenu par la sélection des échos de formation de l'image de la cible en fonction des coordonnées de la région d'impact du faisceau échographique, par rapport à la source échographique ; par l'asservissement du déplacement de l'ensemble des deux sources à partir de signaux dérivés des échos sélectionnés, jusqu'à obtention de ladite coïncidence et par le déclenchement automatique des tirs lorsque cette coïncidence est réalisée.

Suivant un mode d'exécution préféré, ledit balayage échographique est un balayage sectoriel du type B effectué successivement dans deux plans orthogonaux et la sélection des échos est faite, dans chaque plan, en fonction de la position angulaire du faisceau échographique par rapport à l'axe du balayage et de la distance de la région d'impact à la source échographique.

Un second perfectionnement propre à l'invention porte sur la mesure complémentaire décrite dans le brevet susvisé, et consiste à utiliser simultanément la source principale comme émetteur d'un faisceau échographique fixe et la source auxiliaire comme émetteur d'un faisceau échographique à balayage, la source auxiliaire étant alors utilisée comme récepteur des échos formés par réflexion des deux faisceaux, ce qui permet d'obtenir simultanément une image de la cible et une image de la tache focale superposée en surbrillance à l'image de la cible.

L'invention a encore pour objet des dispositifs pour la mise en oeuvre des procédés susvisés.

D'autres particularités, ainsi que les avantages de l'invention, apparaîtront clairement à la lumière de la description ci-après.

Au dessin annexé :

La figure 1 est un schéma de principe d'un dispositif de localisation et de traitement avec poursuite de la cible conforme à un mode d'exécution préféré ; dont

La figure 2 illustre le fonctionnement ;

La figure 3 est un schéma de principe d'un circuit de visualisation de la tache focale ; dont

La figure 4 illustre le fonctionnement.

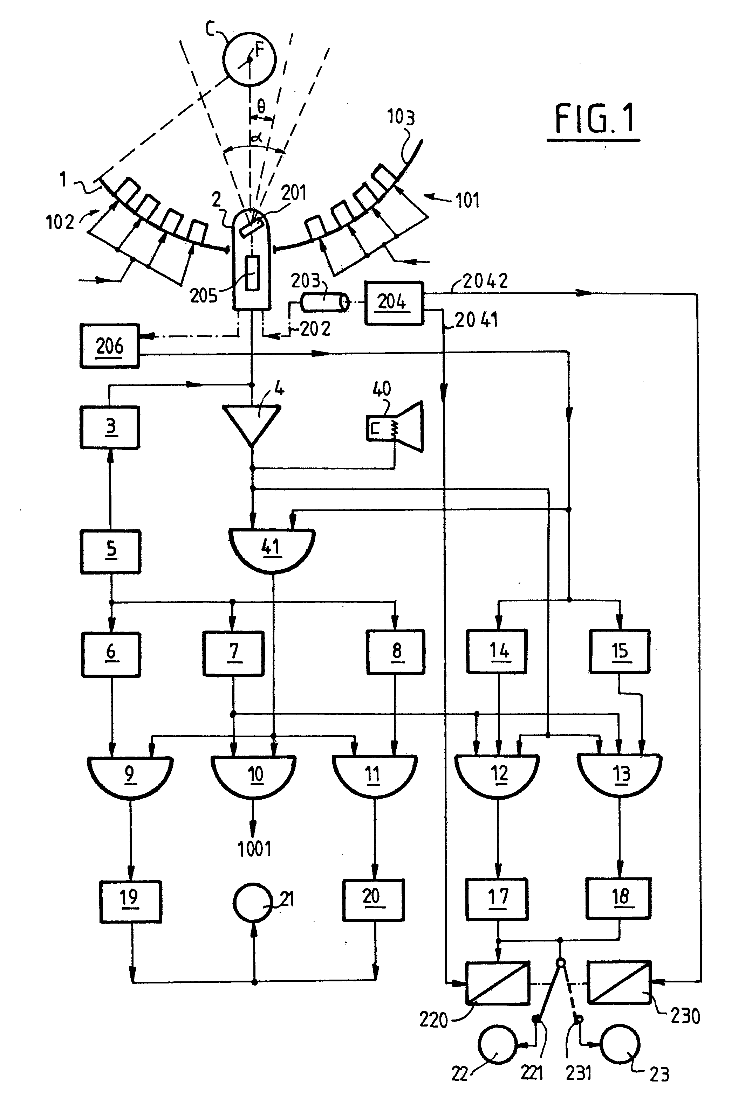

A la figure 1, on a représenté schématiquement un générateur d'ondes ultrasonores de puissance 1 comportant plusieurs groupes de transducteurs piézoélectriques tels que 101, 102 montés sur une calotte sphérique 103 et engendrant un faisceau conique fixe focalisé en F. Il s'agit d'amener le foyer F en coïncidence avec une cible C.

Une sonde d'échographie 2 à balayage sectoriel traverse un orifice ménagé au sommet de la calotte 103 et est disposée suivant l'axe de celle-ci.

Elle comporte une surface émissive et réceptrice oscillante 201 qui émet, lorsqu'elle est excitée par un émetteur d'impulsions électriques 3 synchronisé par une horloge 5, un faisceau étroit d'ultrasons balayant un secteur angulaire $\alpha$ dont l'axe de symétrie se confond avec celui de la calotte. La sonde 2 est montée de façon à pouvoir tourner autour de son axe, tout en étant solidaire des déplacements du générateur 1 suivant trois axes de coordonnées rectangulaires. Ces déplacements orthogonaux sont respectivement commandés, de manière non figurée, par trois moteurs 21 (déplacement suivant l'axe de la coupelle ou "vertical"), 22 (déplacement dans le plan de la figure, ou "longitudinal") et 23 (déplacement perpendiculaire au plan de la figure ou "transversal"). A intervalles réguliers, la sonde 2 est par ailleurs entraînée en rotation autour de son axe de façon à passer, par exemple en moins d'une seconde, d'une première position dans laquelle le faisceau échographique balaye le plan de la figure, à une deuxième position dans laquelle il balaie un plan orthogonal. Ce déplacement angulaire périodique est commandé automatiquement, par un moteur 203 par l'intermédiaire d'un mécanisme de transmission symbolisé par une ligne en trait mixte 202. Un dispositif 204, qui peut comporter deux interrupteurs de fin de course, fournit un signal sur l'une de ses sorties 2041 ou 2042, suivant que le balayage s'effectue dans le plan de la figure ou dans un plan perpendiculaire.

Les échos reçus par la sonde 2 sont amplifiés par un amplificateur de réception 4, puis transmis, par l'intermédiaire d'une porte ET 41, à des portes ET 9, 10 et 11, au moment où un signal indiquant que le faisceau échographique est sensiblement axé sur l'axe de la sonde ($\theta = \varepsilon$) est fourni à la porte 41 par le dispositif 206. Les échos, quand ils existent, c'est-à-dire quand la cible est dans le faisceau de la sonde, sont par ailleurs transmis en permanence à une première entrée des portes ET, 12 et 13 reliée à la sortie de l'amplificateur 4.

Les portes ET 9, 10 et 11 sont respectivement ouvertes par des signaux qui leur sont appliqués par des générateurs de créneaux 6, 7 et 8, respectivement destinés, comme on l'expliquera dans la suite, à effectuer la sélection des échos provenant de surfaces réfléchissantes situées, sur l'axe du dispositif, respectivement au-delà du foyer F, au foyer lui-même, et en deçà du foyer. Ces créneaux, de durée prédéterminée, sont initialisés par les impulsions de synchronisation formées par l'horloge 5.

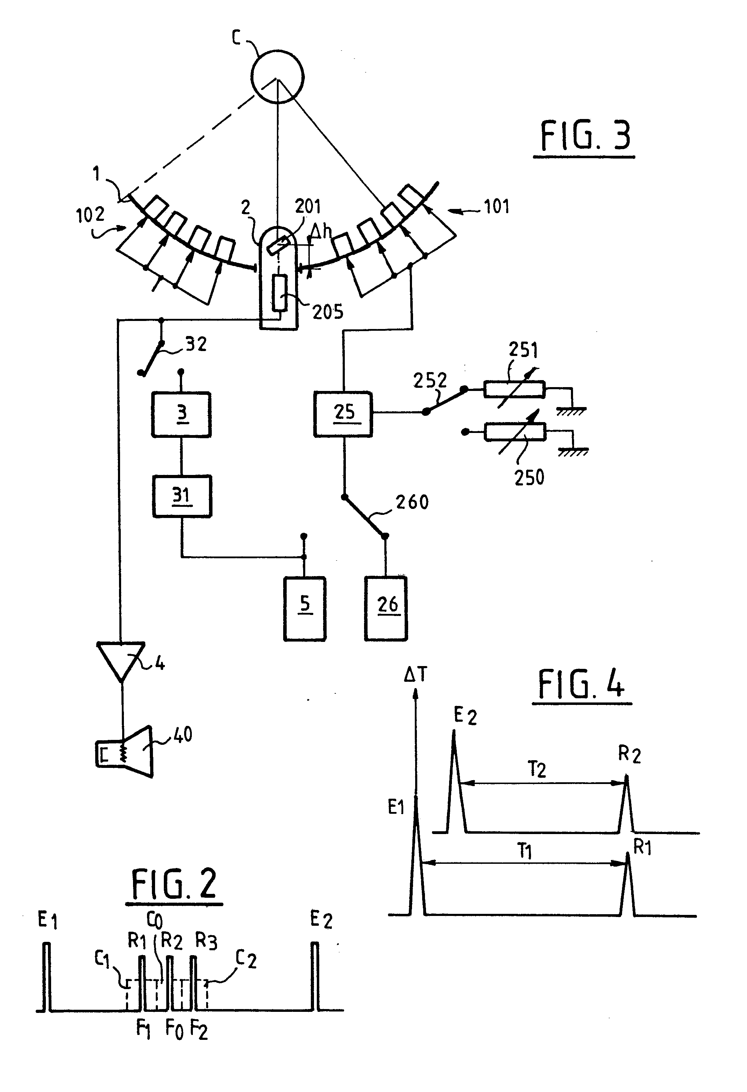

La figure 2 représente deux impulsions d'émission successives $E_1$ et $E_2$ et des échos $R_1$, $R_2$, $R_3$ respectivement situés dans des fenêtres de réception $F_1$, $F_0$, $F_2$ définies par des créneaux $C_1$, $C_0$, $C_2$ synchronisés sur l'impulsion $E_1$ et correspondant à trois plages de distances prédéterminées à partir de la surface émissive de la sonde 2. La plage correspondant à $F_0$ est celle qui contient la tache focale du générateur 1, celle-ci a grossièrement la forme d'un ellipsoïde allongé dont le grand axe coïncide avec celui du générateur 1 ; la plage correspondant à $F_1$ est située en deçà de la tache focale, tandis que la plage qui correspond à $F_2$ est située au-delà.

Lorsqu'un écho est détecté dans la fenêtre $F_2$, il est clair que cela signifie que la cible est située au-delà du foyer dans le sens vertical et il faut donc déplacer le générateur vers le haut pour faire coïncider le foyer avec la cible. A l'inverse, en cas de détection d'un écho dans la fenêtre $F_1$, il faut déplacer le générateur vers le bas.

Les signaux d'échos transmis pendant la durée de ces créneaux fournis par les générateurs 6 et 8, c'est-à-dire correspondant à des échos formés dans les plages $F_2$ et $F_1$ respectivement, sont respectivement appliqués à des circuits 19 et 20 qui commandent la marche du moteur 21 dans le sens approprié pour corriger le décalage vertical de la cible par rapport au foyer en déplaçant le générateur 1.

Lorsque la cible coïncide avec la plage centrale $F_0$ qui contient la tache focale, un écho est transmis par la porte 10 et celle-ci transmet à sa sortie 1001 un signal de déclenchement des tirs. Il n'en est toutefois ainsi que si l'écho a été transmis par la porte 41, donc si le générateur a préalablement été positionné de façon que son axe traverse la cible ($\theta = \varepsilon$).

A cet effet, une deuxième entrée des portes ET 12 et 13 est reliée la sortie du générateur de créneaux 7, tandis qu'une troisième entrée est reliée à l'un ou l'autre de deux comparateurs 14 et 15 eux-mêmes reliés au circuit 206 qui leur fournit à chaque instant une indication numérique de la position auxiliaire $\theta$ du faisceau de balayage de la sonde 2. Lorsque $\theta$ est dans la plage $\Delta\theta + \varepsilon$, le comparateur 14 envoie un niveau logique 1 à la porte 12, tandis que le comparateur 4 envoie un niveau logique 1 à la porte 13 lorsque $\theta$ est dans la plage $\Delta\theta - \varepsilon$. $\Delta\theta$ varie de 0 à une valeur maximum qui définit deux plages d'approche angulaire de la cible, tandis que $\varepsilon$ est une valeur fixe qui correspond avantageusement à la moitié de l'angle sous lequel est vue la tache focale du générateur 1 depuis le centre de la surface émissive de la sonde.

Les portes 12 et 13 sont reliées à deux circuits 17 et 18 qui commandent la marche de l'un ou l'autre des moteurs 22, 23, respectivement dans un sens ou dans l'autre. Le moteur 22 est commandé lorsqu'un relais 220 établit la liaison entre les circuits 17 et 18 et son entrée de commande 221, tandis que le moteur 23 est commandé lorsqu'un relais 230 établit la liaison entre les circuits 17 et 18 et son entrée de commande 231. Ces deux relais sont respectivement commandés par des signaux qui apparaissent aux sorties respectives 2041 et 2042 du dispositif 204, c'est-à-dire lorsque le balayage est effectué dans le plan de la figure ou, respectivement, dans le plan perpendiculaire.

Le moteur 22 tourne ainsi dans un sens ou dans l'autre pour effectuer un déplacement longitudinal du générateur 1 dans un sens propre à amener sa tache focale sous la cible.

Dès que l'information de position angulaire fournie par le circuit 206 sort de la plage des comparateurs, les portes 12 et 13 sont fermées, si bien que le moteur 22 s'arrête, ce qui signifie que la tache focale est sur la cible dans le sens longitudinal ($\theta = \varepsilon$).

Le moteur 23 effectue de la même façon le déplacement transversal du générateur dans un sens ou dans l'autre, qui amènera la tache focale sur la cible dans le sens transversal, et c'est seulement lorsque la coïncidence est obtenue à la fois dans le sens longitudinal et dans le sens transversal que le dispositif 206, correctement agencé à cet effet, indiquera que $\theta = \varepsilon$ et ouvrira la porte 41 pour autoriser le tir.

On observera que le dispositif de la figure 1 définit, dans l'espace qui contient la tache focale, deux groupes, contenus dans deux plans orthogonaux respectifs, de trois plages, à savoir une plage centrale qui encadre étroitement la tache focale dans sa petite dimension, et deux plages d'approche disposées de part et d'autre de la plage centrale. Il définit en outre, dans chacun de ces deux plans, trois plages de distances centrées au sommet du faisceau, à savoir une plage centrale qui encadre étroitement la tache focale dans sa grande dimension et deux plages d'approche disposées de part et d'autre de la plage centrale.

Le procédé mis en oeuvre permet ainsi, à condition qu'un positionnement grossier de la cible ait été préalablement effectué par un procédé de localisation visuelle de préférence du type décrit au brevet susvisé pour l'amener dans les plages d'approche, d'effectuer une poursuite automatique permanente de la cible pour déclencher les tirs avec une grande précision. Cette poursuite sera avantageusement continuée pendant les tirs, le signal de déclenchement à la sortie 1001 correspondant par exemple, à cet effet, à un niveau logique établi à un tant que la cible est atteinte, et dont le passage à zéro arrête les tirs.

Le procédé s'analyse en effet comme un asservissement du déplacement solidaire du radiateur de puissance et de la sonde échographique en fonction des coordonnées de la région d'impact du faisceau échographique sur la cible fixe, avec comme consigne les coordonnées qui correspondent à une position du radiateur pour laquelle son foyer est situé sur la surface de la cible. La sonde se déplaçant en même temps que le radiateur, les coordonnées de la région d'impact du faisceau échographique sur la cible sont en effet liées à la position du radiateur. Pour n'importe quel type de balayage échographique, les coordonnées instantanées peuvent être déterminées à partir de la distance échographique (déterminable d'après le temps de propagation des échos) et de la position du faisceau échographique (connue puisque le balayage est commandé).

Le mode d'exécution préféré décrit est toutefois de mise en oeuvre particulièrement simple et efficace.

A la figure 3, on a représenté schématiquement un circuit supplémentaire du dispositif de la figure 1, destiné à la visualisation de la tache focale. Comme à la figure 1, la synchronisation des impulsions échographiques est assurée par une horloge 5, mais un circuit de retard réglable 31 est interposé entre l'horloge 5 et l'émetteur 3, et ce dernier est connecté à la sonde 2 par l'intermédiaire d'un interrupteur 32 qui permet de mettre en service ou non l'émission échographique. Comme à la figure 1, les circuits de réception et de formation d'image ont été représentés de manière symbolique, sous la forme de l'amplificateur de réception 4 qui attaque l'électrode de commande de luminosité du tube cathodique 40. Par contre, on a figuré l'émetteur 25 qui excite, de manière connue en soi et représenté de façon purement symbolique, les transducteurs du générateur de puissance 1.

La puissance de l'émetteur 21 est réglable (par exemple de 0,5 à 1 Kw en valeur de crête) par des moyens, connus en soi, symbolisés par deux résistances réglables 250-251 reliées à une entrée de commande de l'émetteur 25 par l'intermédiaire d'un commutateur 252.

L'émetteur 25 est, pendant le mode de fonctionnement actuellement décrit et qui est destiné à la visualisation de la tache focale, synchronisé par l'horloge 5 (position 1 du commutateur 260). Il doit être bien compris que, pour son fonctionnement en mode traitement, l'émetteur 25 travaille à puissance non réduite (100 Kw en valeur de crête par exemple). Il est alors synchronisé par une horloge 26, le commutateur 260 étant alors en position 2.

Lorsque, comme indiqué à la figure 3, le commutateur 260 est en position 1 et que le commutateur 252 est sur l'une des deux (ou plusieurs) positions d'émission à puissance réduite (soit 251 par exemple), l'émetteur 25 est synchronisé pour émettre par exemple 256 impulsions pendant l'intervalle de 1/10 de seconde qui sépare normalement deux tirs et est utilisé pour l'échographie. Ces impulsions (telles que $E_1$ figure 4) sont réfléchies sur la cible, qui a été préalablement localisée pour qu'une surface réfléchissante de celle-ci soit située au foyer du radiateur et les échos $R_1$ ainsi formés, et provenant d'une région de la cible coïncidant avec la tache focale du radiateur, sont reçus par la sonde 2 et procurent une image de ladite tache focale, comme on l'a expliqué dans le brevet mentionné. Comme le faisceau est émis par le radiateur fixe, seule la tache focale est clairement visible sur cette image.

Dans le dispositif représenté, il est possible de mettre simultanément en service les émetteurs 25 et 23 (en agissant à la fois sur les commutateurs 260 et 32). La sonde 2 fonctionne alors à la fois comme émetteur échographique et comme récepteur des échos résultant des réflexions des faisceaux simultanément émis par la sonde et le radiateur, si bien que l'image de la tache focale apparaît en surbrillance sur l'image de la cible et de son environnement formée par la sonde. Le réglage de la puissance de l'émetteur 25 permet de doser les luminosités respectives.

Comme le montre la figure 3, les distances respectives du radiateur et de la sonde au foyer sont différentes de $\Delta h$, si bien que (figure 4) les temps $T_1$ et $T_2$ qui séparent les échos respectifs ($R_1$ et $R_2$) des impulsions d'émission correspondantes ($E_1$ et $E_2$) sont différents d'un intervalle $\Delta T$ proportionnel à $\Delta h$. Pour que les échos $R_1$ et $R_2$ coïncident dans le temps, afin d'obtenir la formation simultanée des deux images, on règle le retard fourni par le circuit 31 à la valeur $\Delta T = c\Delta h$, c étant la vitesse de propagation.

Il va de soi que diverses modifications pourront être apportées aux dispositifs décrits et représentés, sans s'écarter de l'esprit de l'invention.

Il doit être bien compris que les procédés et dispositifs décrits s'appliquent aussi bien à l'hyperthermie qu'à la lithotripsie, ou à tout autre traitement par ondes élastiques focalisées requérant la localisation échographique précise de la cible et/ou la visualisation de la tache focale du générateur de l'onde de traitement.

## Revendications

1. Dispositif de localisation et de traitement d'une cible anatomique comportant un générateur de puissance ayant une surface concave de transmission d'un faisceau focalisé fixe d'ondes élastiques de traitement, des moyens de repérage par échographie de la position de la région focale dudit faisceau fixe comportant au moins une sonde échographique agencée pour effectuer un balayage plan avec un faisceau échographique dans la région de l'espace occupé par ledit faisceau focalisé fixe, des moyens de formation d'une image échographique en temps réel de la cible pendant la période de traitement, des moyens d'affichage, sur le support de visualisation de ladite image, d'un repère représentant la position théorique du foyer du faisceau focalisé fixe, et des moyens de déplacement solidaire de ladite surface concave de transmission et de ladite sonde échographique jusqu'à l'obtention de la coïncidence du repère avec l'image de la cible, caractérisé par des moyens de sélection des échos de formation de l'image de la cible en fonction des coordonnées de la région d'impact dudit faisceau échographique par rapport à ladite source échographique, par des moyens d'asservissement dudit déplacement à partir de signaux dérivés des échos sélectionnés jusqu'à obtention de ladite coïncidence et par des moyens de déclenchement automatique de l'émission des ondes de traitement lorsque cette coïncidence est réalisée.

2. Dispositif selon la revendication 1, caractérisé en ce que le balayage du faisceau échographique est un balayage sectoriel du type B effectué successivement dans deux plans orthogonaux et que la sélection des échos est faite, dans chaque plan, en fonction de la position angulaire du faisceau échographique par rapport à l'axe du secteur balayé et de la distance de la région d'impact à la sonde échographique.

3. Dispositif selon la revendication 2, caractérisé en ce qu'il comporte des premiers moyens (6 à 11 et 41), de sélectionner et de séparer les échos formés dans des régions d'impact respectivement situées à une distance qui correspond sensiblement à celle dudit foyer et à des distances inférieure et supérieure, mais correspondant à une dite position angulaire définie par un angle sensiblement nul, des seconds moyens (7 et 12 à 15) de sélectionner et de séparer les échos formés dans des régions d'impact respectivement situées, dans chacun des deux plans de balayage, dans desdites positions angulaires définies par des angles respectivement nul, positif et négatif ; des moyens (206) de fournir auxdits premiers et seconds moyens une information relative à ladite position angulaire, des moyens (204) de faire tourner la sonde échographique autour dudit axe de balayage pour l'amener alternativement, à intervalles réguliers, dans les positions qui correspondent aux deux plans de balayage, des moyens (17-18-220-230-22-23) de commander ledit déplacement pour annuler sensiblement ledit angle, des moyens (19 à 21) de commander ledit déplacement pour que la région d'impact soit située à ladite distance correspondant sensiblement au foyer et des moyens (10) de déclencher l'émission des ondes de traitement lorsque les premiers moyens transmettent un écho correspondant à ladite distance.

4. Dispositif de localisation et de traitement d'une cible anatomique comportant un générateur de puissance ayant une surface concave de transmission d'un faisceau focalisé fixe d'ondes élastiques de traitement, des moyens de repérage par échographie de la position de la région focale dudit faisceau fixe comportant au moins une sonde échographique agencée pour effectuer un balayage plan avec un faisceau échographique dans la région de l'espace occupé par ledit faisceau focalisé fixe, des moyens de formation d'une image échographique en temps réel de la cible pendant la période de traitement, des moyens d'affichage, sur le support de visualisation de ladite image, d'un repère représentant la position théorique du foyer du faisceau focalisé fixe, et des moyens de déplacement solidaire de ladite surface concave de transmission et de ladite sonde échographique jusqu'à l'obtention de la coïncidence du repère avec l'image de la cible, ledit dispositif comportant en outre des moyens de faire émettre audit générateur de puissance, pendant les intervalles entre des impulsions principales qui constituent lesdites ondes élastiques de traitement, une pluralité d'impulsions de contrôle de la tache focale dudit faisceau focalisé fixe, ayant une puissance sensiblement plus réduite que celle des impulsions principales, à la même cadence que celles émises par ladite sonde échographique,

caractérisé par l'émission simultanée, en impulsions à ladite cadence échographique, par le générateur de puissance utilisé en émetteur et par la sonde échographique utilisée en émetteur-récepteur, respectivement d'un faisceau fixe d'ondes élastiques à puissance réduite par rapport à la puissance de traitement et d'un faisceau soumis à un balayage échographique, par la réception des échos correspondant par la source échographique, et par la formation d'une image de la cible et de son environnement, sur laquelle la tache focale de la source de puissance apparaît en surbrillance.

5. Dispositif selon la revendication 4, caractérisé en ce que les impulsions émises par la sonde échographique sont à la même cadence que les impulsions émises par la source de puissance, mais sont retardées par rapport à celles-ci d'un intervalle de temps proportionnel à la différence des distances entre le foyer du générateur de puissance et les surfaces de transmission des deux faisceaux respectifs.

EP 0 310 470 A1

FIG.1

FIG. 3

FIG. 4

FIG. 2

Office européen
des brevets

RAPPORT DE RECHERCHE EUROPEENNE

Numero de la demande

EP 88 40 2236

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | EP-A-0 148 653 (DORY) <br> * En entier * <br> --- | 1 | A 61 B 17/22 |
| A | EP-A-0 169 311 (DORMER SYSTEM) <br> --- | | |
| A | DE-A-3 617 032 (INBAR) <br> --- | | |
| A | EP-A-0 170 416 (MATSUMOTO) <br> ----- | | |

**DOMAINES TECHNIQUES RECHERCHES (Int. Cl.4)**

A 61 B
A 61 F

**Le présent rapport a été établi pour toutes les revendications**

| Lieu de la recherche | Date d'achèvement de la recherche | Examinateur |
|---|---|---|
| LA HAYE | 10-01-1989 | STEENBAKKER J. |